# EUROPEAN PATENT APPLICATION

(11) **EP 1 547 598 A1**
(43) Date of publication of application: **29.06.2005**
(21) Application number: 03733521.3
(22) Date of filing: 20.06.2003
(51) Int. Cl.: A61K 31/5575, A61K 31/4166, A61K 45/00, C07D 233/78, A61P 1/14, A61P 3/04

(54) **NOVEL USES OF PROSTAGLANDIN D sb 2 /sb , PROSTAGLANDIN D sb 2 /sb AGONIST AND PROSTAGLANDIN D sb 2 /sb ANTAGONIST**

(30) Priority: 30.09.2002 JP 2002285637
(71) Applicant: SHIONOGI & CO., LTD., Osaka-shi, Osaka 541-0045 (JP)
(72) Inventor: YOSHIKAWA, Masaaki, Jyoyo-shi, Kyoto 610-0114 (JP); TAKAGI, Kuniko, Kumagaya-shi, Saitama 360-0013 (JP); OHINATA, Kousaku, Uji-shi, Kyoto-fu 611-0011 (JP); INUI, Akio, Kagoshima-ken 892-0838 (JP); ASAKAWA, Akihiro, Kobe-shi, Hyogo 650-0017 (JP); KAKUDO, Shinji, Shionogi & Co., Ltd., Osaka-shi, Osaka 553-0002 (JP)
(74) Representative: Hayes, Adrian Chetwynd
(86) International application number: PCT/JP2003/007837
(87) International publication number: WO 2004/030674

(57) **Abstract**

The present invention provides novel use of PGD₂, a PGD₂ agonist and a PGD₂ antagonist. More specifically, the present invention provides uses of PGD₂ and a PGD₂ agonist as eating promoter and use of a PGD₂ antagonist as an eating inhibitor.

## Description

### TECHNICAL FIELD

The present invention relates to novel uses of PGD₂ (prostaglandin D₂) related substances. Specifically, the present invention relates to uses of PGD₂ and a PGD₂ agonist for the stimulation of food intake and use of a PGD₂ antagonist for the inhibition of food intake.

### BACKGROUND ART

PGD₂ is a major prostanoid that is produced in mast cells in which it is produced through PGG₂ and PGH₂ from arachidonic acid by the action of cyclooxygenase activated by immunological or unimmunological stimulation (e.g. Urade Y, Kitahama K, Ohishi H et al., Proc Natl Acad Sci USA. 90, 9070-9074 (1993); Beuckmann CT, Lazarus M, Gerashchenko D et al., J Comp Neurol 428, 62-78 (2000)). PGD₂ is believed to be a causative agent of allergic diseases such as allergic rhinitis and allergic conjunctivitis because it shows various physiological and pathological activities such as induction of nasal obstruction, vascular dilation, and eosinotaxis etc. Moreover, a large quantity of PGD₂ is also released from macrophages, and therefore, PGD₂ may play a role in causing an inflammatory response independent of allergy. Furthermore, PGD₂ is the most abundant in the central nervous system (CNS) of mammals, including human, and known to be involved in various central actions, such as sleep induction, decrease of body temperature and modulation of pain response (e.g., Hayaishi O., FASEB J 5, 2575-2581 (1991), Hayaishi O., J Appl Physiol 92, 863-868 (2002), Urade Y and Hayaishi O., Biochim Biophys Acta 1436, 606-615 (1999), Eguchi N, Minami T, Shirafuji N et al., Proc Natl Acad Sci USA. 96, 726-730 (1999)). However, any effect of PGD₂ on the regulation of food intake has not been described.

PGD₂ antagonists are known to be useful in the improvement of conditions due to excessive production of PGD₂, particularly as drugs for treating diseases in which mast cell dysfunction is involved, for example, systemic mastocytosis and disorder of systemic mast cell activation as well as for tracheal contraction, asthma, allergic rhinitis, allergic conjunctivitis, urticaria, ischemic reperfusion injury, antiinflammatory drugs and drugs for treating atopic dermatitis. However, any effect of PGD₂ antagonist on food intake disorder has not been described.

### DISCLOSURE OF INVENTION

The present invention provides novel uses of PGD₂ related substances in the regulation of food intake. The present invention is based on the discovery that PGD₂ has an orexigenic action, and PGD₂ antagonist has an anorexigenic action.

That is, the present invention provides:
(1) a pharmaceutical composition comprising an effective amount of a PGD₂ related substance for the regulation of food intake;
(2) a pharmaceutical composition comprising an effective amount of PGD₂ or a PGD₂ agonist for the stimulation of food intake;
(3) a pharmaceutical composition comprising an effective amount of a PGD₂ antagonist for the inhibition of food intake;
(4) the pharmaceutical composition of (3) wherein the PGD₂ antagonist is a compound of the formula: 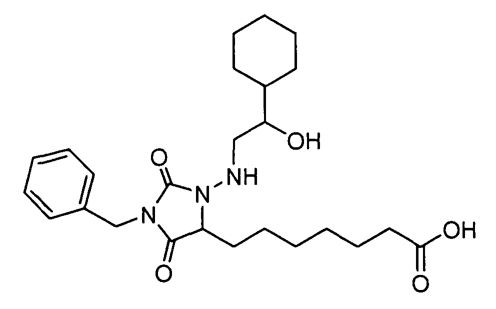 or a pharmaceutically acceptable salt thereof, or a solvate thereof;
(5) the pharmaceutical composition of (3) wherein the PGD₂ antagonist is a compound of the formula (I): 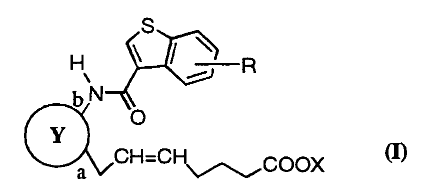 wherein 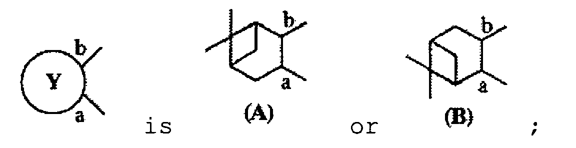 R is hydrogen, alkyl, alkoxy, halogen, hydroxy, acyloxy or optionally substituted arylsulfonyloxy;
   X is hydrogen or alkyl; and
   the double bond on the alpha-chain has E configuration or Z configuration
   or a pharmaceutically acceptable salt thereof, or a solvate thereof;
(6) the pharmaceutical composition of (5) wherein the PGD₂ antagonist is a compound of the formula (IB): 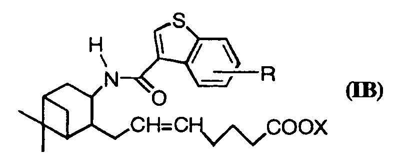 wherein
   R and X are as defined above; and
   the double bond on the alpha-chain has E configuration or Z configuration
   or a pharmaceutically acceptable salt thereof, or a solvate thereof;
(7) the pharmaceutical composition of (5) wherein the PGD₂ antagonist is a compound of the formula (IA-a): 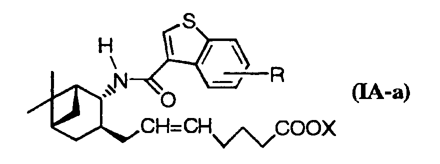 wherein
   R and X are as defined above; and
   the double bond on the alpha-chain has E configuration or Z configuration
   or a pharmaceutically acceptable salt thereof, or a solvate thereof;
(8) the pharmaceutical composition of (7) wherein the PGD₂ antagonist is a compound of the formula (IA-a1): 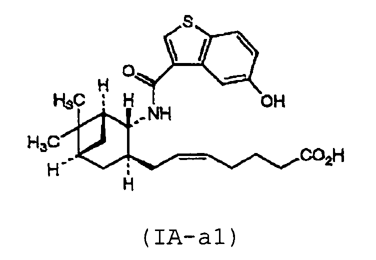 or a pharmaceutically acceptable salt thereof, or a solvate thereof;
(9) a method for regulating food intake in a mammal including human, comprising administering to the mammal an effective amount of a PGD₂ related substance;
(10) a method for stimulating food intake in a mammal including human, comprising administering to the mammal an effective amount of PGD₂ or a PGD₂ agonist;
(11) a method for inhibiting food intake in a mammal including human, comprising administering to the mammal an effective amount of a PGD₂ antagonist;
(12) the method of (11) wherein the PGD₂ antagonist is a compound of the formula: 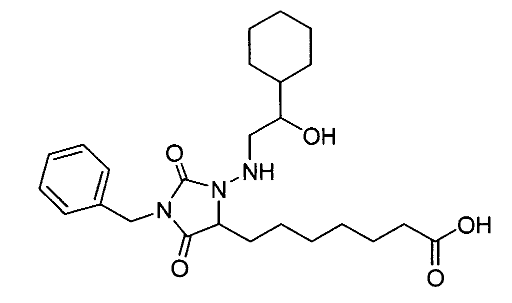 or a pharmaceutically acceptable salt thereof, or a solvate thereof;
(13) the method of (11) wherein the PGD₂ antagonist is a compound of the formula (I): 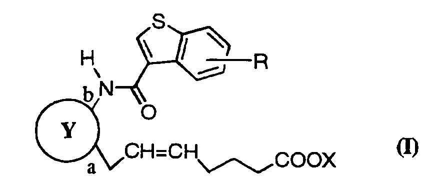 wherein 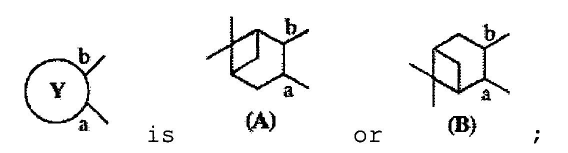 R is hydrogen, alkyl, alkoxy, halogen, hydroxy, acyloxy or optionally substituted arylsulfonyloxy;
   X is hydrogen or alkyl; and
   the double bond on the alpha-chain has E configuration or Z configuration
   or a pharmaceutically acceptable salt thereof, or a solvate thereof;
(14) the method of (13) wherein the PGD₂ antagonist is a compound of the formula (IB): 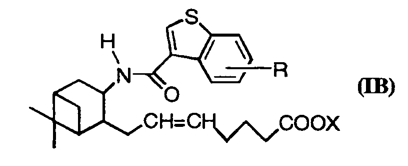 wherein
   R and X are as defined above; and
   the double bond on the alpha-chain has E configuration or Z configuration
   or a pharmaceutically acceptable salt thereof, or a solvate thereof;
(15) the method of (13) wherein the PGD₂ antagonist is a compound of the formula (IA-a): 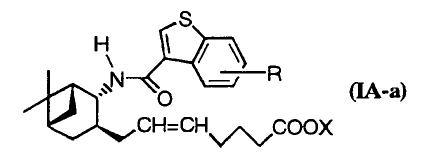 wherein
   R and X are as defined above; and
   the double bond on the alpha-chain has E configuration or Z configuration
   or a pharmaceutically acceptable salt thereof, or a solvate thereof;
(16) the method of (15) wherein the PGD₂ antagonist is a compound of the formula (IA-a1): 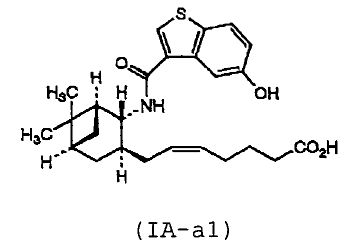 or a pharmaceutically acceptable salt thereof, or a solvate thereof;
(17) use of a PGD₂ related substance for the manufacturing of a medicament for the regulation of food intake;
(18) use of PGD₂ or a PGD₂ agonist for the manufacturing of a medicament for the stimulation of food intake;
(19) use of a PGD₂ antagonist for the manufacturing of a medicament for the inhibition of food intake;
(20) the use of (19), wherein the PGD₂ antagonist is a compound of the formula: 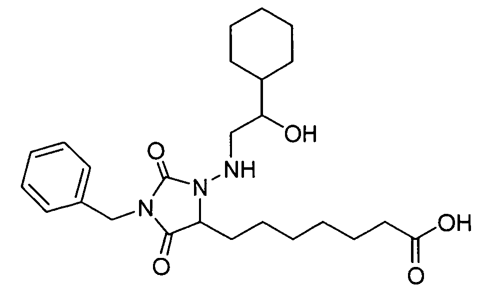 or a pharmaceutically acceptable salt thereof, or a solvate thereof;
(21) the use of (19) wherein the PGD₂ antagonist is a compound of the formula (I): 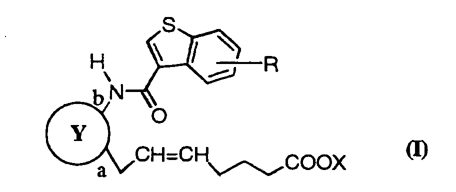 wherein 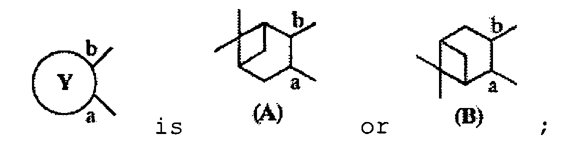 R is hydrogen, alkyl, alkoxy, halogen, hydroxy, acyloxy or optionally substituted arylsulfonyloxy;
   X is hydrogen or alkyl; and
   the double bond on the alpha-chain has E configuration or Z configuration
   or a pharmaceutically acceptable salt thereof, or a solvate thereof;
(22) the use of (21) wherein the PGD₂ antagonist is a compound of the formula (IB): 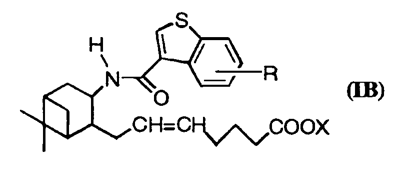 wherein
   R and X are as defined above; and
   the double bond on the alpha-chain has E configuration or Z configuration
   or a pharmaceutically acceptable salt thereof, or a solvate thereof;
(23) the use of (21) wherein the PGD₂ antagonist is a compound of the formula (IA-a): 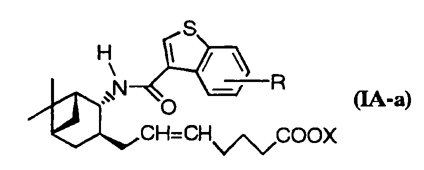 wherein
   R and X are as defined above;
   the double bond on the alpha-chain has E configuration or Z configuration
   or a pharmaceutically acceptable salt thereof, or a solvate thereof;
(24) the use of (23) wherein the PGD₂ antagonist is a compound of the formula (IA-a1): 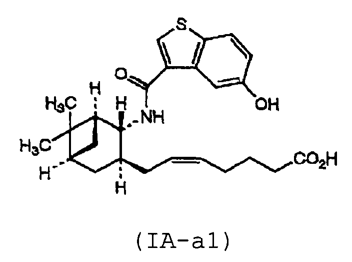 or a pharmaceutically acceptable salt thereof, or a solvate thereof.

### BRIEF DESCRIPTION OF THE DRAWINGS

Fig 1 depicts a graph showing food intake of mice after intracerebroventricular (ICV) administration of PGD₂. The vertical scale indicates cumulative food intake (g), and the horizontal scale indicates time (min.) after the administration of a sample. The group that received the vehicle (artificial cerebrospinal fluid, ACSF) alone was used as a control. Cumulative food intake was expressed as the mean ± SEM of 12-16 mice on each group. ** p<0.01, * p<0.05 versus control (Bonferroni's t-test).

Fig. 2 depicts a graph comparing the food intake two hours after the ICV administration between the group that received PGD₂ alone and that received PGD₂ and a PGD₂ antagonist. The vertical scale indicates cumulative food intake (g). The group that received the vehicle (ACSF) was used as a control. Cumulative food intake was expressed as the mean ± SEM of 6-7 mice on each group.
* p<0.05 versus another group (Bonferroni's t-test).

Fig. 3 depicts a graph showing the food intake of mice after the ICV administration of PGD₂ antagonist. The vertical scale indicates cumulative food intake (g), and the horizontal scale indicates time after administration of the sample. The group that received a vehicle (ACSF) was used as a control.

### BEST MODE FOR CARRYING OUT THE INVENTION

The term "prostaglandin D₂ related substance", as used herein, refers to prostaglandin D₂ (PGD₂), PGD₂ agonists and PGD₂ antagonists, as well as substances that result in any effect on the action of PGD₂, such as those that play a role in the PGD₂ biosynthesis.

The term "regulation of food intake", as used herein, refers to an action to result in any effect on food intake of a mammal, including human. It is contemplated to mean both an action to stimulate food intake and an action to inhibit food intake.

The PGD₂ agonist of the invention is a substance that can bind to PGD₂ receptor selectively and elicit a physiological action of the PGD₂ receptor.

Examples of the PGD₂ agonist include but not limited to prostaglandin derivatives such as those disclosed in WO01/19790, WO01/12596 and the like.

The PGD₂ antagonist of the invention is a substance that can bind selectively to PGD₂ receptor and inhibit the physiological action of the PGD₂ receptor.

Examples of such PGD₂ antagonist include but not limited to indole derivatives such as those disclosed in WO01/66520, carbazol derivatives such as those disclosed in WO01/79169, bicyclo derivatives such as those disclosed in WO02/36583, bicyclo derivatives such as those disclosed in WO01/94309, bicyclo derivatives such as those disclosed in WO00/53573, bicyclo derivatives such as those disclosed in WO97/853, hydantoin derivatives such as those disclosed in European Patent Publication No.284202, benzothiophene derivatives such as those disclosed in WO98/25919.

A preferred PGD₂ antagonist of the invention has a high PGD₂ antagonistic activity and a high selectivity. For example, a preferred antagonist has a PGD₂ binding inhibitory activity (IC₅₀ value) of 1000 µM or less, 1000 nM or less or especially 10 nM or less. The PGD₂ antagonistic activity can be calculated in accordance with conventional method as disclosed in WO98/25919.

A preferred PGD₂ antagonist of the invention is (±)-3-benzyl-5-(6-carboxyhexyl)-1-(2-cyclohexyl-2-hydroxyethylamino)hydantoin, which is represented by the formula

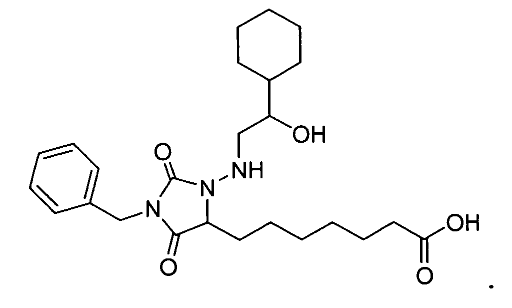

The compound can be prepared by the process disclosed in European Patent Publication No.284202 and also commercially available (e.g., Sigma-Aldrich, BW A868C). Pharmaceutically acceptable salts of the compound or solvates thereof may also be used as a PGD₂ antagonist of the invention.

Also, a compound of the following formula, which is as disclosed in WO98/25919, is preferable as a PGD₂ antagonist of the invention. Specifically, it is a compound represented by the formula (I): 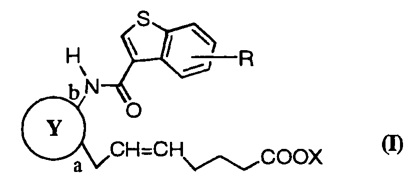 wherein 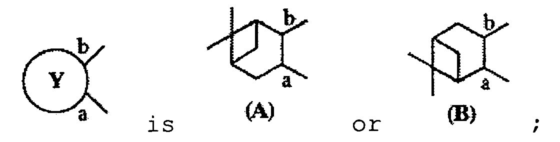 R is hydrogen, alkyl, alkoxy, halogen, hydroxy, acyloxy or optionally substituted arylsulfonyloxy;
X is hydrogen or alkyl;
the double bond on the alpha-chain has E configuration or Z configuration
or a pharmaceutically acceptable salt thereof, or a solvate thereof; and specifically a compound represented by the formula (IB): 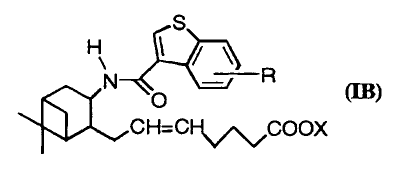 wherein
R and X are as defined above, and
the double bond on the alpha-chain has E configuration or Z configuration
or a pharmaceutically acceptable salt thereof, or a solvate thereof; and specifically a compound represented by the formula (IA-a): 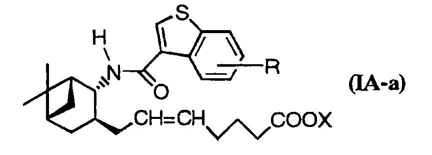 wherein
R and X are as defined above, and
the double bond on the alpha-chain has E configuration or Z configuration
or a pharmaceutically acceptable salt thereof, or a solvate thereof.

More preferably, the PGD₂ antagonist of the invention is a compound represented by the formula (IA-a1): 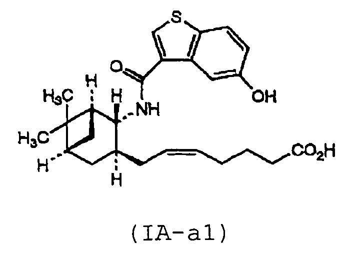 or a pharmaceutically acceptable salt thereof, or a solvate thereof.

Examples of pharmaceutically acceptable salts of the above compounds include those formed with an alkali metal such as lithium, sodium or potassium, an alkali earth metal such as calcium, an organic base such as tromethamine, trimethylamine, triethylamine, 2-aminobutane, t-butylamine, diisopropylethylamine, n-butylmethylamine, cyclohexylamine, dicyclohexylamine, N-isopropylcyclohexylamine, furfurylamine, benzylamine, methylbenzylamine, dibenzylamine, N,N-dimethylbenzylamine, 2-chlorobenzylamine, 4-methoxybenzylamine, 1-naphthylmethylamine, diphenylbenzylamine, triphenylamine, 1-naphthylamine, 1-anthramine, 2-anthramine, dehydroabiethylamine, N-methylmorpholine or pyridine, an amino acid such as lysine, or arginine, and the like. These salts can be formed in a conventional manner. Hydrates of the compound of formula (I) may have any number of water molecules coordinated with the compound at the optional proportion.

The compounds represented by the formula (I) may have E configuration or Z configuration, the bond binding to the bicyclic ring can represent R configuration or S configuration, and therefore, may include respective stereoisomers (diastereomers, epimers, enantiomers, and the like), racemates and mixtures thereof.

All the compounds represented by the above formulae can be prepared according to the process disclosed in WO98/25919.

The PGD₂ or the PGD₂ agonist of the invention has an orexigenic action and is useful in the treatment of cibophobia and decreased appetite in cachexia and the like.

The PGD₂ antagonist of the invention has an anorexigenic action and is useful in the treatment of hyperorexia and obesity and the like.

The PGD₂ related substance of the invention can be formulated into ordinary formulations for oral and parenteral administration to use in the treatments. The pharmaceutical composition of the invention can be in the form for oral and parenteral administration such as tablets, capsules, granules, powders, syrup and the like; those for parenteral administration such as injectable solution or suspension for intravenous, intramuscular or subcutaneous injection, inhalant, eye drops, nasal drops, suppositories, or percutaneous formulations such as ointment, patches poultices and the like.

These formulations can be prepared using appropriate carriers, excipients, solvents, and bases known to one ordinary skilled in the art. In case of tablets, they are prepared by compressing or molding an active ingredient together with auxiliary components. Examples of auxiliary components include pharmaceutically acceptable excipients such as binders (e.g., cornstarch), fillers (e.g., lactose, microcrystalline cellulose), disintegrants (e.g., starch sodium glycolate) or lubricants (e.g., magnesium stearate). Tablets may be coated appropriately. Liquid formulations such as syrups, solutions, or suspensions may contain suspending agents (e.g., methyl cellulose), emulsifiers (e.g., lecithin), preservatives and the like. For injectable formulations, it may be in the form of solution or suspension, or oil- or water-based emulsion, which may contain suspension stabilizers, dispersants or the like. Percutaneous formulations such as ointment, patches poultices can be formulated using an aqueous vehicle (water, lower alcohols, polyols) or a lipophilic vehicle (higher fatty acid esters (isopropyl myristate), lipophilic alcohols).

Appropriate dosage of the PGD₂ related substance varies depending on the administration route, conditions of the patient, age, body weight, sex, and if any, the kind of drug(s) used together and the like, but it should be determined conclusively by the physician. Generally, in the case of oral administration, the daily dosage would be between 0.01-100 mg, preferably 0.1-10 mg, more preferably 0.1-1 mg, per kg body weight. For parenteral administration, the daily dosage can be between 0.001-100 mg, preferably 0.001-1 mg, more preferably 0.001-0.1 mg, per kg body weight. The daily dosage may be administered in 1-4 divisions.

In the present invention, it was revealed that the concentration of PGD₂ to induce orexigenic action was lower than the sadative one. Accordingly, the PGD₂ related substance of the invention is preferably used at a lower concentration than that as previously used for different purposes.

| Formulation 1: tablet | |
|---|---|
| Active Ingredient | 40.0mg |
| Hydroxypropylcellulose | 3.6mg |
| Magnesium stearate | 0.4mg |
| Cornstarch | 18.0mg |
| Lactose | 58.0mg |
| Total | 120.0mg |

| Formulation 2: ointment | |
|---|---|
| Active Ingredient | 0.1g |
| Liquid paraffin | 1.5g |
| White petrolatum | 18.4g |
| Total | 20.0g |

| Formulation 3: injectable solution | |
|---|---|
| Active Ingredient | 100mg |
| Saturated fatty acid glyceride | 1000ml |

A solution consisting of the above ingredients are injected into a patient, generally at a rate of 1ml/min.

### EXAMPLES

The following Examples are provided to further illustrate the present invention. According to the following procedure, the PGD₂ related substances of the invention were examined for their activity in the regulation of food intake.

### Preparation and administration of the test compound:

Male ddY mice (Japan SLC, Inc., Shizuoka, Japan) at 7 weeks old were individually housed in a cage under regulated conditions (22°C on a 12 hr light-dark cycle), and food and water were available ad libitum. The test compound was dissolved in artificial cerebrospinal fluid (ACSF; 138.9 mM NaCl, 3.4 mM KCI, 1.3 mM CaCl₂, 4.0 mM NaHCO₃, 0.6 mM NaH₂PO₄ 5.6 mM glucose, pH 7.4) to prepare a solution of the test compound. The solution was administered via ICV injection as described in Ohinata K, Inui A, Asakawa A et al., FEBS Lett 473, 207-211 (2000); Ohinata K, Inui A, Asakawa A et al., Peptides 22, 589-595 (2001); Asakawa A, Inui A, Kaga T et al., Gastroenterology 120, 337-345 (2001). Specifically, a non-fasted mouse was anesthetized with sodium pentobarbital (80-85 mg/kg, i.p.) and placed in a stereotaxic instrument. A guide cannula was implanted for injection into the third cerebral ventricle (24-gauge, Safelet-Cas, Nipro, Osaka, Japan), and a test compound was injected seven days after. 4µl of a solution of the test compound or vehicle (ACSF) alone was injected into the third cerebral ventricle. For the control group, only the vehicle (ACSF) was injected.

### Measurement of food intake:

The weight of food pellets was measured before and after ICV injection and cumulative food intake was calculated.

### Statistical analysis:

Values were expressed as the mean ± SEM. Analysis of variance (ANOVA) followed by Bonferroni's t-test was used to assess differences among groups. P values less than 0.05 were considered significant.

### Experiment 1: orexigenic action of PGD₂

Mice were administered PGD₂ (Sigma-Aldrich, 0.2-20 µg/mouse) via ICV injection and investigated for change in food intake. Results are shown in Fig.1. With compared to the control group, the group that received 0.2µg or 2µg of PGD₂ showed significant increase in food intake at 60 and 120 min after the injection, indicating that PGD₂ stimulates food intake. In the group that received 20µg of PGD₂, the food intake was temporally inhibited shortly after the injection, but the food intake was increased thereafter. In this group, reduction in locomotion, i.e., sedation, was observed shortly after the PGD₂ administration, and the locomotion was gradually increased thereafter. Accordingly, the temporal inhibition of food intake might be secondary to sedation. This suggests that the concentration of PGD₂ to induce the orexigenic action was lower than that to induce the sedative action.

### Experiment 2: involvement of PGD₂ receptor in orexigenic action

The groups, one of which was administered PGD₂ alone and the other received combined administration of PGD₂/PGD₂ antagonist, were used to investigate the effect of PGD₂ antagonist on the orexigenic action of PGD₂. PGD₂ antagonist, (±) 3-benzyl-5-(6-carboxyhexyl)-1-(2-cyclohexyl-2-hydroxyethylamino)hydantoin (BW A868C, Sigma-Aldrich) was used. As shown in Fig. 2, combined administration of PGD₂/PGD₂ antagonist reduced the food intake to the same level as observed in the group that received vehicle alone, indicating that the PGD₂ antagonist inhibited the orexigenic action of PGD₂ completely. The result suggests that the PGD₂ receptor is involved in the orexigenic action because BWA868C is a specific antagonist of PGD₂ receptor.

### Experiment 3: anorexigenic action of PGD₂ antagonist

### (1) Anorexigenic action of (±) 3-benzyl-5-(6-carboxyhexyl)-1-(2-cyclohexyl-2-hydroxyethylamino)hydantoin

The PGD₂ antagonist used in Experiment 2, i.e., (±) 3-benzyl-5-(6-carboxyhexyl)-1-(2-cyclohexyl-2-hydroxyethylamino)hydantoin (BW A868C, Sigma-Aldrich), was used to investigate anorexigenic action in mice. The compound was administered to mice via ICV injection (0.72 and 7.2µg/mouse) as described in Experiment 1, and the food intake was compared with the group that received the vehicle (ACSF).

As shown in Fig. 3, inhibition of food intake was observed in the group that received the test compound, with compared to that received vehicle.

### (2) Anorexigenic action of (Z)-7-[(1R,2R,3S,5S)]-2-(5-hydroxybenzo[b]thiophene-3-ylcarbonylamino)-10-norpinane-3-yl]heptane-5-enonic acid (formula IA-a1)

The PGD₂ antagonist, (Z)-7-[(1R,2R,3S,5S)]-2-(5-hydroxybenzo[b]thiophene-3-ylcarbonylamino)-10-norpinane-3-yl]heptan-5-enonic acid (formula IA-a1) was used to investigate anorexigenic action in mice. The sodium salt of this compound was administered to SD rat, fasted for 24 hours, via ICV injection as described above (dosage: 0.1, 1, 10, 100µg/rat), and the food intake was compared with the group that received vehicle (5% DMSO in saline). The results are shown in Table 1.

**Table 1:**

| Compound (IA-a1) (ICV administration) | | | | | |
|---|---|---|---|---|---|
| | n | Cululative Food intake (g) | | | |
| | | 1 hr. | 2 hr. | 4 hr. | 24 hr. |
| Saline (5% DMSO) | 17 | 7.01 ± 0.31 | 8.31 ± 0.40 | 10.11 ± 0.58 | 32.54 ± 1.13 |
| Compund (IA-a1) (Na salt) 100 µg | 19 | 5.18 ± 0.47* | 6.15 ± 0.50** | 6.86 ± 0.49** | 25.71 ± 1.30** |
| | | | | | |

| | n | Changes in body weght (g) | | | |
|---|---|---|---|---|---|
| | | 1 hr. | 2 hr. | 4 hr. | 24 hr. |
| Saline (5% DMSO) | 17 | 10.85 ± 0.74 | 13.00 ± 0.91 | 13.54 ± 1.45 | 23.18 ± 2.73 |
| Compund (IA-a1) (Na salt) 100 µg | 19 | 0.89 ± 0.79** | 2.89 ± 1.28** | 1.07 ± 1.47** | 7.20 ± 2.60** |

Also, another group of SD rat was administered the test compound via intraperitoneal administration (30mg/kg) and the food intake was compared with the group that received vehicle (5% DMSO in saline). The results are shown in Table 2.

**Table 2:**

| Compound ( IA-a1 ) (i.p. administration) | | | | | |
|---|---|---|---|---|---|
| | n | Cululative Food intake (g) | | | |
| | | 1 hr. | 2 hr. | 4 hr. | 24 hr. |
| Saline (5% DMSO) | 8 | 5.86 ± 0.32 | 6.91 ± 0.63 | 8.64 ± 0.95 | 35.11 ± 0.60 |
| Compund (IA-a1) (Na salt) 30mg/kg | 8 | 2.38 ± 0.41** | 4.30 ± 0.67* | 7.85 ± 0.66 | 32.05 ± 1.09 |

Compared to the control group, as shown in Tables 1 and 2, inhibition of the food intake was observed in both the groups that received the test compound interperitoneally and intracerebroventricularly. Also, in the group that received intracerebroventricular administration, inhibiting effect on body weight gain was observed (see, Table 1, the lower column).

### INDUSTRIAL APPLICABILITY

The present invention showed that the PGD₂ related substances have an activity to regulate food intake. That is, PGD₂ showed orexigenic action and the PGD₂ antagonists showed anorexigenic action. Also, the PGD₂ antagonists showed inhibition of body weight gain. Thus, PGD₂ and a PGD₂ agonist of the invention are useful in the treatment of cibophobia and decreased appetite in cachexia etc. and useful in the manufacturing of a pharmaceutical composition for the stimulation of food intake. Additionally, PGD₂ antagonists of the invention are useful in the treatment of hyperorexia and obesity etc. and useful in the manufacturing of a pharmaceutical composition for the inhibition of food intake.

## Claims

1. A pharmaceutical composition comprising an effective amount of a prostaglandin D₂ related substance for the regulation of food intake.

2. A pharmaceutical composition comprising an effective amount of prostaglandin D₂ or a prostaglandin D₂ agonist for the stimulation of food intake.

3. A pharmaceutical composition comprising an effective amount of a prostaglandin D₂ antagonist for the inhibition of food intake.

4. The pharmaceutical composition according to Claim 3 wherein said prostaglandin D₂ antagonist is a compound of the formula: 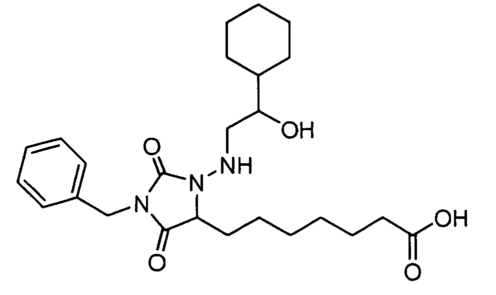 or a pharmaceutically acceptable salt thereof, or a solvate thereof.

5. The pharmaceutical composition according to Claim 3 wherein said prostaglandin D₂ antagonist is a compound of the formula (I): 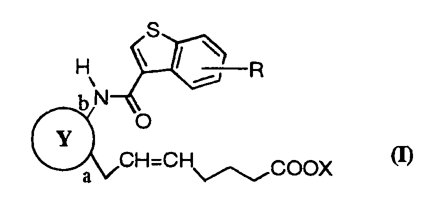 wherein 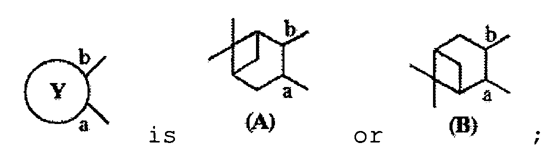 R is hydrogen, alkyl, alkoxy, halogen, hydroxy, acyloxy or optionally substituted arylsulfonyloxy;
X is hydrogen or alkyl; and
the double bond on the alpha-chain has E configuration or Z configuration
or a pharmaceutically acceptable salt thereof, or a solvate thereof.

6. The pharmaceutical composition according to Claim 5 wherein said prostaglandin D₂ antagonist is a compound of the formula (IB): 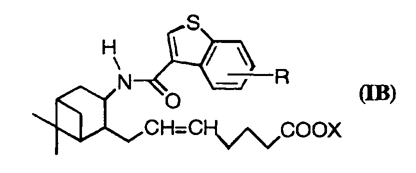 wherein
R and X are as defined above; and
the double bond on the alpha-chain has E configuration or Z configuration
or a pharmaceutically acceptable salt thereof, or a solvate thereof.

7. The pharmaceutical composition according to Claim 5 wherein said prostaglandin D₂ antagonist is a compound of the formula (IA-a) : 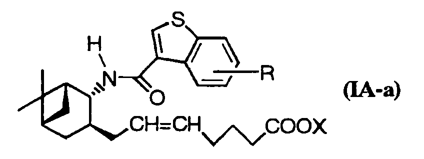 wherein
R and X are as defined above; and
the double bond on the alpha-chain has E configuration or Z configuration
or a pharmaceutically acceptable salt thereof, or a solvate thereof.

8. The pharmaceutical composition according to Claim 7 wherein said prostaglandin D₂ antagonist is a compound of the formula (IA-a1): 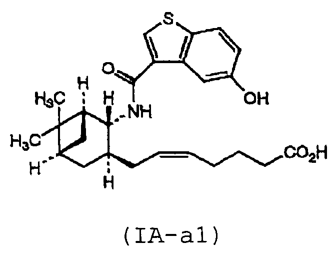 or a pharmaceutically acceptable salt thereof, or a solvate thereof.

9. A method for regulating food intake in a mammal including human, comprising administering to said mammal an effective amount of prostaglandin D₂ related substance.

10. A method for stimulating food intake in a mammal including human, comprising administering to said mammal an effective amount of prostaglandin D₂ or a prostaglandin D₂ agonist.

11. A method for inhibiting food intake in a mammal including human, comprising administering to said mammal an effective amount of a prostaglandin D₂ antagonist.

12. The method according to Claim 11 wherein said prostaglandin D₂ antagonist is a compound of the formula: 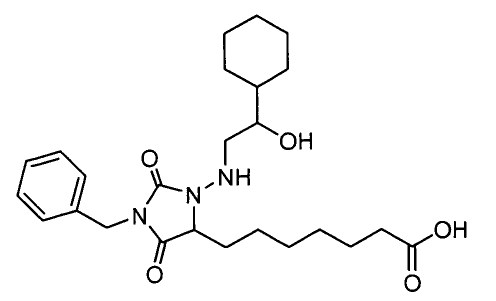 or a pharmaceutically acceptable salt thereof, or a solvate thereof.

13. The method according to Claim 11 wherein said prostaglandin D₂ antagonist is a compound of the formula (I): 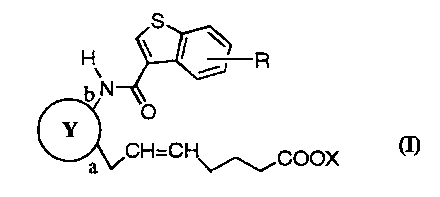 wherein 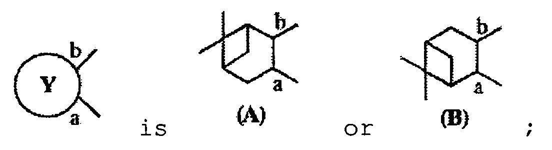 R is hydrogen, alkyl, alkoxy, halogen, hydroxy, acyloxy or optionally substituted arylsulfonyloxy;
X is hydrogen or alkyl; and
the double bond on the alpha-chain has E configuration or Z configuration
or a pharmaceutically acceptable salt thereof, or a solvate thereof.

14. The method according to Claim 13 wherein said prostaglandin D₂ antagonist is a compound of the formula (IB): 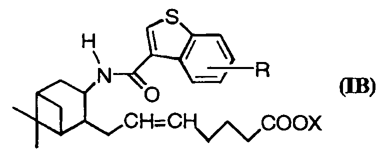 wherein
R and X are as defined above; and
the double bond on the alpha-chain has E configuration or Z configuration
or a pharmaceutically acceptable salt thereof, or a solvate thereof.

15. The method according to Claim 13 wherein said prostaglandin D₂ antagonist is a compound of the formula (IA-a) : 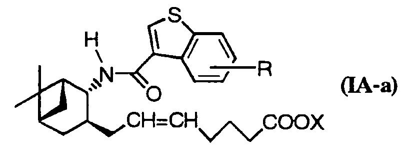 wherein
R and X are as defined above; and
the double bond on the alpha-chain has E configuration or Z configuration
or a pharmaceutically acceptable salt thereof, or a solvate thereof.

16. The method according to Claim 15 wherein said prostaglandin D₂ antagonist is a compound of the formula (IA-a1): 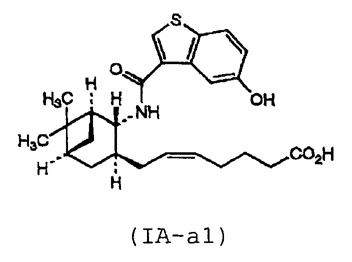 or a pharmaceutically acceptable salt thereof, or a solvate thereof.

17. Use of prostaglandin D₂ related substance for the manufacturing of a medicament for the regulation of food intake.

18. Use of prostaglandin D₂ or a prostaglandin D₂ agonist for the manufacturing of a medicament for the stimulation of food intake.

19. Use of prostaglandin D₂ antagonist for the manufacturing of a medicament for the inhibition of food intake.

20. The use according to claim 19, wherein said prostaglandin D₂ antagonist is a compound of the formula: 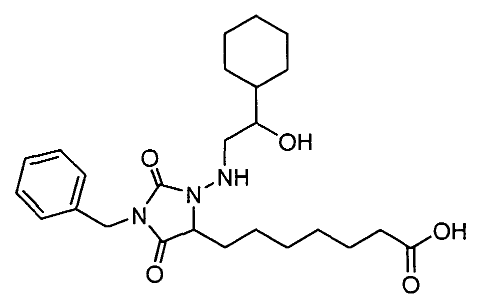 or a pharmaceutically acceptable salt thereof, or a solvate thereof.

21. The use according to Claim 19 wherein said prostaglandin D₂ antagonist is a compound of the formula (I): 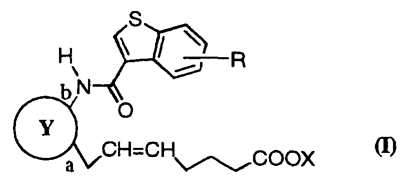 wherein 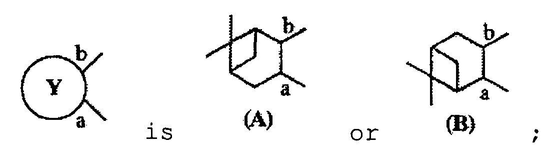 R is hydrogen, alkyl, alkoxy, halogen, hydroxy, acyloxy or optionally substituted arylsulfonyloxy;
X is hydrogen or alkyl; and
the double bond on the alpha-chain has E configuration or Z configuration
or a pharmaceutically acceptable salt thereof, or a solvate thereof.

22. The use according to Claim 21 wherein said prostaglandin D₂ antagonist is a compound of the formula (IB) : 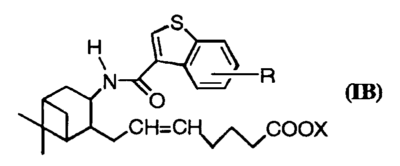 wherein
R and X are as defined above; and
the double bond on the alpha-chain has E configuration or Z configuration
or a pharmaceutically acceptable salt thereof, or a solvate thereof.

23. The use according to Claim 21 wherein said prostaglandin D₂ antagonist is a compound of the formula (IA-a) : 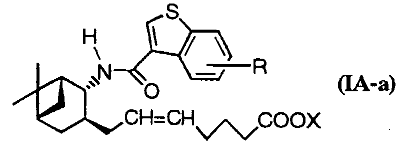 wherein
R and X are as defined above;
the double bond on the alpha-chain has E configuration or Z configuration
or a pharmaceutically acceptable salt thereof, or a solvate thereof.

24. The use according to Claim 23 wherein said prostaglandin D₂ antagonist is a compound of the formula (IA-a1): 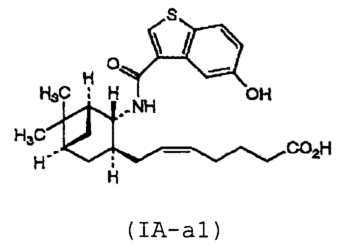 or a pharmaceutically acceptable salt thereof, or a solvate thereof.
